(19) Europäisches Patentamt
European Patent Office
Office européen des brevets

(11) EP 1 088 814 A1

(12) EUROPÄISCHE PATENTANMELDUNG

(43) Veröffentlichungstag:
**04.04.2001 Patentblatt 2001/14**

(51) Int. Cl.[7]: **C07C 311/09**, C07C 303/38

(21) Anmeldenummer: **00120189.6**

(22) Anmeldetag: **22.09.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **29.09.1999 DE 19946673**
**09.11.1999 DE 19953638**

(71) Anmelder: **MERCK PATENT GmbH**
**64293 Darmstadt (DE)**

(72) Erfinder:
- **Schmidt, Michael Dr.**
  **64342 Seeheim-Jugenheim (DE)**
- **Sartori, Peter Prof. Dr.**
  **47985 Rheinberg (DE)**
- **Ignatiev, Nikolai Dr.**
  **47058 Duisburg (DE)**

# (54) Fluorierte Sulfonamide als schwer entflammbare Lösungsmittel zum Einsatz in elektrochemischen Zellen

(57) Die Erfindung betrifft fluorierte Sulfonamide als schwer entflammbare Lösungsmittel für Elektrolyten zum Einsatz in elektrochemischen Zellen.

EP 1 088 814 A1

## Beschreibung

Die Erfindung betrifft fluorierte Sulfonamide als schwer entflammbare Lösungsmittel für Elektrolyten zum Einsatz in elektrochemischen Zellen.

Lithium-Ionen-Batterien gehören zu den aussichtsreichsten Systemen für mobile Anwendungen. Die Einsatzgebiete reichen dabei von hochwertigen Elektronikgeräten (z.B. Mobiltelefone, Camcorder) bis hin zu Batterien für Kraftfahrzeuge mit Elektroantrieb.

Diese Batterien bestehen aus Kathode, Anode, Separator und einem nichtwäßrigen Elektrolyt. Als Kathode werden typischerweise $Li(MnMe_z)_2O_4$, $Li(CoMe_z)O_2$, $Li(CoNi_xMe_z)O_2$ oder andere Lithium-Interkalations und Insertions-Verbindungen verwendet. Anoden können aus Lithium-Metall, Kohlenstoffen, Graphit, graphitischen Kohlenstoffen oder andere Lithium-Interkalations und Insertions-Verbindungen oder Legierungsverbindungen bestehen. Als Elektrolyt werden Lösungen mit Lithiumsalzen wie $LiPF_6$, $LiBF_4$, $LiClO_4$, $LiAsF_6$, $LiCF_3SO_3$, $LiN(CF_3SO_2)_2$ oder $LiC(CF_3SO_2)_3$ und deren Mischungen in aprotischen Lösungsmitteln verwendet.

Eine Vielzahl von Additiven zur Anwendung in Lithium-Ionen-Batterien werden in der Literatur erwähnt. Beispielsweise werden in EP 0759641 und US 5776627 organische aromatische Verbindungen wie Biphenyl, substituierte Thiophene und Furane, in EP 0746050 und EP 0851524 substituierte Anisol-, Mesithylen- und Xylolderivate dem Elektrolyten zugesetzt um die Sicherheit der Batterie im Fall einer Überladung zu erhöhen. Zum gleichen Zweck werden in US 5753389 organische Carbonate als Additive verwendet. Zur Verbesserung der Zyklenstabilität werden in EP 0856901 organische Boroxine zugegeben. All diese Additive haben jedoch einige entscheidende Nachteile. Organische Substanzen, wie sie in den hier genannten Schriften verwendet werden, besitzen im allgemeinen tiefe Flammpunkte und niedrige Explosionsgrenzen.

| Additiv | Explosionsgrenze [%] | Flammpunkt [°C] |
|---|---|---|
| Thiophen | 1,5-12 | -9 |
| Anisol | 0,34-6,3 | 43 |
| Mesitylen | 1-6 | 54 |
| Furan | 2,3-14,3 | -35 |

Als Elektrolytflüssigkeiten verwendet man nach dem derzeitigen Stand der Technik bevorzugt Lösungsmittelgemische von mindestens zwei Komponenten. Das Gemisch muß mindestens eine stark polare Komponente enthalten, die aufgrund ihrer Polarität auf Salze stark dissoziierend wirkt. Beispiele für diese polaren Komponenten sind Ethylen- oder Propylencarbonat. Da diese hochpolaren Lösungsmittel meist eine sehr hohe Viskosität aufweisen, werden dem Elektrolyten im allgemeinen niedrigviskose Solventien als „Verdünner" zugesetzt. Typische Vertreter sind 1,2-Dimethoxyethan, Dimethylcarbonat oder Diethylcarbonat, die in einem Anteil zwischen 30-70% zugesetzt werden. Ein gravierender Nachteil dieser niedrigviskosen Lösungsmittel liegt in ihren tiefen Flammpunkten und hohen Flüchtigkeit.

Da bei elektrochemischer Anwendung von Elektrolytlösungen und in noch weit stärkerem Maße beim Auftreten von Fehlern (Kurzschluß, Überladen) stets eine Erwärmung eintritt, erhöht dies das Risiko der Entzündung des Elektrolyten.

Zur Erhöhung der Sicherheit können Kathoden- und Anodenraum durch eine mikroporöse Separatormembran getrennt werden. Weiterhin kann der Einbau von Überdrucksicherungen, die auf Gasentwicklung beim Überladen reagieren, die Sicherheit dieser Zellen erhöhen.

Es werden flammhemmende phosphor- und halogenhaltige Zusätze empfohlen, die sich allerdings häufig negativ auf die Leistungscharakteristik der Batterien auswirken.

All diese Maßnahmen können jedoch nicht ausschließen, daß bei Betriebsstörungen der leichtflüchtige und leichtentzündliche „Verdünner" letztlich doch entflammt. Brennendes Lithium reagiert nicht nur mit Wasser, sondern auch mit Kohlendioxid sehr heftig.

Aufgabe der vorliegenden Erfindung ist es deshalb, Additive zur Verfügung zu stellen, deren Flüchtigkeit gering ist und deren Flammpunkte relativ hoch liegen, die physikalisch und chemisch stabil und mit anderen geeigneten Lösungsmitteln ausreichend mischbar sind und ein gutes Leitfahigkeitsverhalten aufweisen.

Die erfindungsgemäße Aufgabe wird gelöst durch Verbindungen der allgemeinen Formel

$$X\text{-}(CYZ)_m\text{-}SO_2N(CR^1R^2R^3)_2 \qquad (I)$$

mit

| | |
|---|---|
| X | H, F, Cl, $C_nF_{2n+1}$, $C_nF_{2n-1}$, $(SO_2)_kN(CR^1R^2R^3)_2$ |
| Y | H, F, Cl |
| Z | H, F, Cl |
| $R^1$, $R^2$, $R^3$ | H und/oder Alkyl, Fluoralkyl, Cycloalkyl |
| m | 0-9 und falls X=H, m≠0 |
| n | 1-9 |
| k | 0, falls m=0 und k=1, falls m=1-9. |

Die Verbindungen der Formel (I) können in elektrochemischen Zellen wie Superkondensatoren und primären und sekundären Lithiumbatterien, insbesondere als Lösungsmittel, eingesetzt werden.

Es wurde gefunden, daß die Verbindungen der Formel (I) schwer entflammbar sind. Damit kann die Gefahr der Entzündung beim Auftreten von Fehlern verringert werden.

Überraschend wurde gefunden, daß die Verbindungen der Formel (I) eine hohe elektrochemische Stabilität aufweisen. Versuche haben gezeigt, daß ein Elektrolyt, enthaltend eine Verbindung der Formel (I) und gängige Lösungsmittel (z.B. EC, DMC) sowie ein typisches Leitsalz (z.B. $LiPF_6$), erst ab einem Potential von ca. 5,5 V gegen $Li/Li^+$ oxidativ zersetzt wird.

Es wurde gefunden, daß die Verbindungen der Formel (I) mit den gängigen Lösungsmitteln mischbar sind. Es konnte keine Phasentrennung bzw. Kristallisation des Leitsalzes beobachtet werden.

Die Verbindungen der Formel (I) können auch in Mischungen, mit einem Anteil zwischen 1 und 100%, vorzugsweise zwischen 10% und 50% mit gängigen Lösungsmitteln wie z.B. EC, DMC, PC und DEC in Elektrolyten verwendet werden.

Als Elektrolyte können Lösungen von $LiPF_6$, $LiBF_4$, $LiClO_4$, $LiAsF_6$, $LiCF_3SO_3$, $LiN(CF_3SO_2)_2$ oder $LiC(CF_3SO_2)_3$ und deren Mischungen in aprotischen Lösungsmitteln wie EC, DMC, PC, DEC, BC, VC, Cyclopentanone, Sulfolane, DMS, 3-Methyl-1,3-oxazolidine-2-on, DMC, DEC, γ-Butyrolacton, EMC, MPC, BMC, EPC, BEC, DPC, 1,2-Diethoxymethan, THF, 2-Methyltetrahydrofuran, 1,3-Dioxolan, Methylacetat, Ethylacetat und deren Mischungen verwendet werden.

Die Elektrolyte können auch organische Isocyanate (DE 199 44 603) zur Herabsetzung des Wassergehaltes enthalten. Ebenso können die Elektrolyte organische Alkalisalze (DE 199 10 968) als Additiv enthalten. Geeignet sind Alkaliborate der allgemeinen Formel

$$Li^+ B^-(OR^1)_m(OR^2)_p$$

worin,

| | |
|---|---|
| m und p | 0, 1, 2, 3 oder 4 mit m+p=4 und |
| $R^1$ und $R^2$ | gleich oder verschieden sind, |

gegebenenfalls durch eine Einfach- oder Doppelbindung direkt miteinander verbunden sind,

jeweils einzeln oder gemeinsam die Bedeutung eines aromatischen oder aliphatischen Carbon-, Dicarbon- oder Sulfonsäurerestes haben, oder

jeweils einzeln oder gemeinsam die Bedeutung eines aromatischen Rings aus der Gruppe Phenyl, Naphthyl, Anthracenyl oder Phenanthrenyl, der unsubstituiert oder ein- bis vierfach durch A oder Hal substituiert sein kann, haben oder

jeweils einzeln oder gemeinsam die Bedeutung eines heterocyclischen aromatischen Rings aus der Gruppe Pyridyl, Pyrazyl oder Bipyridyl, der unsubstituiert oder ein- bis dreifach durch A oder Hal substituiert sein kann, haben oder

jeweils einzeln oder gemeinsam die Bedeutung einer aromatischen Hydroxysäure aus der Gruppe aromatischer Hydroxy-Carbonsäuren oder aromatischer Hydroxy-Sulfonsäuren, der unsubstituiert oder ein- bis bis vierfach durch A oder Hal substituiert sein kann,

haben und

Hal F, Cl oder Br und

A Alkyl mit 1 bis 6 C-Atomen, das ein- bis dreifach halogeniert sein kann, bedeuten. Ebenso geeignet sind Alkalialkoholate (DE 9910968) der allgemeinen Formel

$$Li^{+}\ OR^{-}$$

worin R

die Bedeutung eines aromatischen oder aliphatischen Carbon-, Dicarbon- oder Sulfonsäurerestes hat, oder

die Bedeutung eines aromatischen Rings aus der Gruppe Phenyl, Naphthyl, Anthracenyl oder Phenanthrenyl, der unsubstituiert oder ein- bis vierfach durch A oder Hal substituiert sein kann, hat oder

die Bedeutung eines heterocyclischen aromatischen Rings aus der Gruppe Pyridyl, Pyrazyl oder Bipyridyl, der unsubstituiert oder ein- bis dreifach durch A oder Hal substituiert sein kann, hat oder

die Bedeutung einer aromatischen Hydroxysäure aus der Gruppe aromatischer Hydroxy-Carbonsäuren oder aromatischer Hydroxy-Sulfonsäuren, der unsubstituiert oder ein- bis vierfach durch A oder Hal substituiert sein kann,

hat und

Hal F, Cl, oder Br, und

A Alkyl mit 1 bis 6 C-Atomen, das ein- bis dreifach halogeniert sein kann.

Auch Verbindungen der allgemeinen Formel

$$[([R^1\ (CR^2R^3)_k]_l A_x)_y Kt]^{+}\ {}^{-}N(CF_3)_2$$

wobei

Kt N, P, As, Sb, S, Se

A N, P, P(O), O, S, S(O), $SO_2$, As, As(O), Sb, Sb(O)

$R^1$, $R^2$ und $R^3$

gleich oder verschieden

H, Halogen, substituiertes und/oder unsubstituiertes Alkyl $C_nH_{2n+1}$, substituiertes und/oder unsubstituiertes Alkenyl mit 1-18 Kohlenstoffatomen und einer oder mehreren Doppelbindungen, substituiertes und/oder unsubstituiertes Alkinyl mit 1-18 Kohlenstoffatomen und einer oder mehreren Dreifachbindungen, substituiertes und/oder unsubstituiertes Cycloalkyl $C_mH_{2m-1}$, ein- oder mehrfach substituiertes und/oder unsubstituiertes Phenyl, substituiertes und/oder unsubstituiertes Heteroaryl,

A kann in verschiedenen Stellungen in $R^1$, $R^2$ und/oder $R^3$ eingeschlossen sein,

Kt kann in cyclischen oder heterocyclischen Ring eingeschlossen sein,

die an Kt gebundenen Gruppen können gleich oder verschieden sein

mit

n 1-18

m 3-7

k 0, 1-6

l 1 oder 2 im Fall von x=1 und 1 im Fall x=0

x 0,1

y 1-4

bedeuten, können enthalten sein (DE 9941566). Das Verfahren zur Herstellung der Verbindungen ist dadurch gekennzeichnet, daß ein Alkalisalz der allgemeinen Formel

$$D^+ \ ^-N(CF_3)_2$$

mit $D^+$ ausgewählt aus der Gruppe der Alkalimetalle in einem polaren organischen Lösungsmittel mit einem Salz der allgemeinen Formel

$$[([R^1(CR^2R^3)_k]_lA_x)_yKt]^+ \ ^-E$$

wobei

Kt, A, $R^1$, $R^2$, $R^3$, k, l, x und y die oben angegebene Bedeutung haben und

$^-E$ $F^-$, $Cl^-$, $Br^-$ ,$I^-$, $BF_4^-$, $ClO_4^-$, $AsF_6^-$, $SbF_6^-$ oder $PF_6^-$

bedeutet, umgesetzt wird.

Auch Lithiumkomplexsalze der Formel

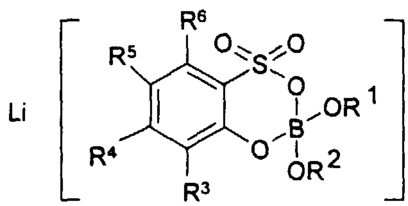

wobei

$R^1$ und $R^2$ gleich oder verschieden sind, gegebenenfalls durch eine Einfach- oder Doppelbildung direkt miteinander verbunden sind, jeweils einzeln oder gemeinsam die Bedeutung eines aromatischen Rings aus der Gruppe Phenyl, Naphthyl, Anthracenyl oder Phenanthrenyl, der unsubstituiert oder ein- bis sechsfach durch Alkyl ($C_1$ bis $C_6$), Alkoxygruppen ($C_1$ bis $C_6$) oder Halogen (F, Cl, Br) substituiert sein kann, haben,

oder jeweils einzeln oder gemeinsam die Bedeutung eines aromatischen heterozyklischen Rings aus der Gruppe Pyridyl, Pyrazyl oder Pyrimidyl, der unsubstituiert oder ein- bis vierfach durch Alkyl ($C_1$ bis $C_6$), Alkoxygruppen ($C_1$ bis $C_6$) oder Halogen (F, Cl, Br) substituiert sein kann, haben,

oder jeweils einzeln oder gemeinsam die Bedeutung eines aromatischen Rings aus der Gruppe Hydroxylbenzoecarboxyl, Hydroxylnaphthalincarboxyl, Hydroxylbenzoesulfonyl und Hydroxylnaphthalinsulfonyl, der unsubstituiert oder ein- bis vierfach durch Alkyl ($C_1$ bis $C_6$), Alkoxygruppen ($C_1$ bis $C_6$) oder Halogen (F, Cl, Br) substituiert sein

kann, haben,

$R^3$-$R^6$ können jeweils einzeln oder paarweise, gegebenenfalls durch eine Einfach- oder Doppelbindung direkt miteinander verbunden, folgende Bedeutung haben:

1. Alkyl ($C_1$ bis $C_6$),Alkyloxy ($C_1$ bis $C_6$) oder Halogen (F, Cl, Br)
2. ein aromatischer Ring aus den Gruppen

   Phenyl, Naphthyl, Anthracenyl oder Phenanthrenyl, der unsubstituiert oder ein- bis sechsfach durch Alkyl ($C_1$ bis $C_6$), Alkoxygruppen ($C_1$ bis $C_6$) oder Halogen (F, Cl, Br) substituiert sein kann,

   Pyridyl, Pyrazyl oder Pyrimidyl, der unsubstituiert oder ein- bis vierfach durch Alkyl ($C_1$ bis $C_6$), Alkoxygruppen ($C_1$ bis $C_6$) oder Halogen (F, Cl, Br) substituiert sein kann,

die über folgendes Verfahren (DE 199 32 317) dargestellt werden

a) 3-, 4-, 5-, 6-substituiertes Phenol in einem geeigneten Lösungsmittel mit Chlorsulfonsäure versetzt wird,

b) das Zwischenprodukt aus a) mit Chlortrimethylsilan umgesetzt, filtriert und fraktioniert destilliert wird,

c) das Zwischenprodukt aus b) mit Lithiumtetramethanolat-borat(1-), in einem geeigneten Lösungsmittel umgesetzt und daraus das Endprodukt isoliert wird, können im Elektrolyten enthalten sein.

Auch Elektrolyte mit Komplexsalzen der allgemeinen Formel (DE 199 51 804)

$$M^{X+}[EZ]^{y-}_{x/y}$$

worin bedeuten:

x, y 1, 2, 3, 4, 5, 6

$M^{X+}$ ein Metallion

E eine Lewis-Säure, ausgewählt aus der Gruppe $BR^1R^2R^3$, $AlR^1R^2R^3$, $PR^1R^2R^3R^4R^5$, $AsR^1R^2R^3R^4R^5$, $VR^1R^2R^3R^4R^5$,

$R^1$ bis $R^5$ gleich oder verschieden, gegebenenfalls durch eine Einfach- oder Doppelbildung direkt miteinander verbunden sind, jeweils einzeln oder gemeinsam die Bedeutung

eines Halogens (F, Cl, Br),

eines Alkyl- oder Alkoxyrestes ($C_1$ bis $C_8$) der teilweise oder vollständig durch F, Cl, Br substituiert sein kann,

eines, gegebenenfalls über Sauerstoff gebundenen aromatischen Rings aus der Gruppe Phenyl, Naphthyl, Anthracenyl oder Phenanthrenyl, der unsubstituiert oder ein- bis sechsfach durch Alkyl ($C_1$ bis $C_8$) oder F, Cl, Br substituiert sein kann

eines, gegebenenfalls über Sauerstoff gebundenen aromatischen heterocyclischen Rings aus der Gruppe Pyridyl, Pyrazyl oder Pyrimidyl, der unsubstituiert oder ein- bis vierfach durch Alkyl ($C_1$ bis $C_8$) oder F, Cl, Br substituiert sein kann, haben können und
Z $OR^6$, $NR^6R^7$, $CR^6R^7R^8$, $OSO_2R^6$, $N(SO_2R^6)(SO_2R^7)$, $C(SO_2R^6)(SO_2R^7)(SO_2R^8)$, $OCOR^6$, wobei

$R^6$ bis $R^8$ gleich oder verschieden sind, gegebenenfalls durch eine Einfach- oder Doppelbindung direkt miteinander verbunden sind, jeweils einzeln oder gemeinsam die Bedeutung

eines Wasserstoffs oder die Bedeutung wie $R^1$ bis $R^5$ haben, hergestellt durch Umsetzung von einem

entsprechenden Bor- oder Phosphor-Lewis-Säure-Solvenz-Adukt mit einem Lithium- oder Tetraalkylammonium-Imid, -Methanid oder -Triflat, können verwendet werden.

Auch Boratsalze (DE 199 59 722) der allgemeinen Formel

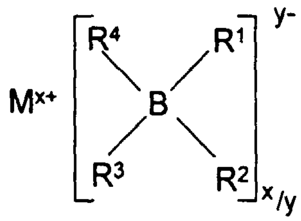

worin bedeuten:

| | |
|---|---|
| M | ein Metallion oder Tetraalkylammoniumion |
| x,y | 1, 2, 3, 4, 5 oder 6 |
| $R^1$ bis $R^4$ | gleich oder verschieden, gegebenenfalls durch eine Einfach- oder Doppelbindung direkt miteinander verbundener Alkoxy- oder Carboxyreste ($C_1$-$C_8$) können enthalten sein. Hergestellt werden diese Boratsalze durch Umsetzung von Lithiumtetraalkoholatborat oder einem 1:1 Gemisch aus Lithiumalkoholat mit einem Borsäureester in einem aprotischen Lösungsmittel mit einer geeigneten Hydroxyl- oder Carboxylverbindung im Verhältnis 2:1 oder 4:1. |

Die erfindungsgemäßen Verbindungen können auch in Elektrolyte eingesetzt werden, die Lithiumfluoralkylphosphate der folgenden Formel enthalten,

$$Li^+[PF_x(C_yF_{2y+1-z}H_z)_{6-x}]^-$$

worin

$1 \leq x \leq 5$

$3 \leq y \leq 8$

$0 \leq z \leq 2y + 1$

bedeuten und die Liganden ($C_yF_{2y+1-z}H_z$) gleich oder verschieden sein können, wobei die Verbindungen der allgemeinen Formel,

$$Li^+[PF_a(CH_bF_c(CF_3)_d)_e]^-$$

in der a eine ganze Zahl von 2 bis 5, b = 0 oder 1, c = 0 oder 1, d = 2 und

e eine ganze Zahl von 1 bis 4 bedeuten, mit den Bedingungen, daß b und c nicht gleichzeitig jeweils = 0 bedeuten und die Summe aus a + e gleich 6 ist und die Liganden ($CH_bF_c(CF_3)_d$) gleich oder verschieden sein können, ausgenommen sind (DE 100 089 55). Das Verfahren zur Herstellung von Lithiumfluoralkylphosphaten ist dadurch gekennzeichnet, daß wenigstens eine Verbindung der allgemeinen Formel

$$H_mP(C_nH_{2n+1})_{3-m} \quad (III),$$

$$OP(C_nH_{2n+1})_3 \quad (IV),$$

$$Cl_mP(C_nH_{2n+1})_{3-m} \quad (IV),$$

$$F_mP(C_nH_{2n+1})_{3-m} \quad (VI),$$

$$Cl_oP(C_nH_{2n+1})_{5-o} \quad (VII),$$

$$F_oP(C_nH_{2n+1})_{5-o} \quad (VIII),$$

in denen jeweils

$0 < m < 2$, $3 < n < 8$ und $0 < o < 4$ bedeutet

durch Elektrolyse in Fluorwasserstoff fluoriert wird, das so erhaltene Gemisch der Fluorierungsprodukte durch Extraktion, Phasentrennung und/oder Destillation aufgetrennt wird, und das so erhaltene fluorierte Alkylphosphoran in einem aprotischen Lösungsmittel oder Lösungsmittelgemisch unter Feuchtigkeitsausschluß mit Lithiumfluorid umgesetzt wird, und das so erhaltene Salz nach den üblichen Methoden gereinigt und isoliert wird.

Die erfindungsgemäßen Verbindungen können auch in Elektrolyten eingesetzt werden, die Salze der Formel

$$Li[P(OR^1)_a(OR^2)_b(OR^3)_c(OR^4)_dFe]$$

worin $0 < a+b+c+d \leq 5$ und $a+b+c+d+e=6$ gilt, und $R^1$ bis $R^4$ unabhängig voneinander Alkyl-, Aryl- oder Heteroarylreste sind, wobei mindestens zwei von $R^1$ bis $R^4$ durch eine Einfach- oder Doppelbindung direkt miteinander verbunden sein können, enthalten (DE 100 16801). Dargestellt werden die Verbindungen durch Umsetzung von Phosphor (V)-Verbindungen der allgemeinen Formel

$$P(OR^1)_a(OR^2)_b(OR^3)_c(OR^4)_dF_e$$

worin $0 < a+b+c+d \leq 5$ und $a+b+c+d+e=5$ gilt, und $R^1$ bis $R^4$ die oben angegebenen Bedeutungen haben mit Lithiumfluorid in Gegenwart eines organischen Lösungsmittels.

Die erfindungsgemäßen Verbindungen können in Elektrolyte für elektrochemische Zellen eingesetzt werden, die Anodenmaterial, bestehend aus beschichteten Metallkernen, ausgewählt aus der Gruppe Sb, Bi, Cd, In, Pb, Ga und Zinn oder deren Legierungen, enthalten (DE 100 16 024). Das Verfahren zur Herstellung dieses Anodenmaterials ist dadurch gekennzeichnet, daß

a) eine Suspension oder ein Sol des Metall- oder Legierungskerns in Urotropin hergestellt wird,

b) die Suspension mit Kohlenwasserstoffen mit $C_5$-$C_{12}$ emulgiert werden,

c) die Emulsion auf die Metall- oder Legierungskerne aufgefällt werden und

d) durch Temperung des Systems die Metallhydroxide bzw. -oxihydroxide in das entsprechende Oxid übergeführt werden. Die erfindungsgemäßen Verbindungen können auch in Elektrolyte für elektrochemische Zellen eingesetzt werden, mit Kathoden aus gängigen Lithium-Interkalations und Insertionsverbindungen aber auch mit Kathodenmaterialien, die aus Lithium-Mischoxid-Partikel bestehen, die mit einem oder mehreren Metalloxiden (DE 199 22 522) beschichtet sind, indem die Partikel in einem organischen Lösungsmittel suspendiert werden, die Suspension mit einer Lösung einer hydrolisierbaren Metallverbindung und einer Hydrolyselösung versetzt und danach die beschichteten Partikel abfiltriert, getrocknet und gegebenenfalls calciniert werden. Sie können auch aus Lithium-Mischoxid-Partikel bestehen, die mit einem oder mehreren Polymeren (DE 199 46 066) beschichtet sind, erhalten durch ein Verfahren, bei dem die Partikel in einem Lösungsmittel suspendiert werden und anschließend die beschichteten Partikel abfiltriert, getrocknet und gegebenenfalls calciniert werden. Ebenso können die erfindungsgemäßen Verbindungen in Systemen mit Kathoden eingesetzt werden, die aus Lithium-Mischoxid-Partikeln bestehen, die mit Alkalimetallverbindungen und Metalloxiden ein- oder mehrfach beschichtet sind (DE 100 14 884). Das Verfahren zur Herstellung dieser Materialien ist dadurch gekennzeichnet, daß die Partikel in einem organischen Lösungsmittel suspendiert werden, eine Alkalimetallsalzverbindung suspendiert in einem organischen Lösungsmittel zugegeben wird, Metalloxide gelöst in einem organischen Lösungsmittel zugegeben werden, die Suspension mit einer Hydrolyselösung versetzt wird und anschließend die beschichteten Partikel abfiltriert, getrocknet und calciniert werden.

Gegenstand der Erfindung sind somit Elektrolyte, welche Verbindungen der Formel (I) enthalten.

Gegenstand der Erfindung sind ferner elektrochemische Zellen, insbesondere primäre und sekundäre Lithiumbatterien und Superkondensatoren, welche im wesentlichen aus einem entsprechenden Elektrolyten sowie Kathode, Anode und Separator bestehen.

Die Verbindungen der allgemeinen Formel (I) in Mischungen mit gängigen Leitsalzen weisen eine gute Leitfähigkeit auf.

Nachfolgend wird ein allgemeines Beispiel der Erfindung näher erläutert.

Herstellung der Verbindungen der Formel (I)

Dimethylamin wird in einem geeigneten Lösungsmittel in einer Apparatur mit Rührer und Kühlung vorgelegt. Als Lösungsmittel sind organische Lösungsmittel, beispielsweise Diethylether oder Chloroform, geeignet.

Unter Kühlung auf Temperaturen zwischen -30°C und 0°C und Rühren werden teil- oder perfluorierte Alkylsulfonylfluoride zugegeben. Danach wird die Reaktionslösung auf Temperaturen zwischen Raumtemperatur und 40°C erwärmt. Das Lösungsmittel wird abdestilliert.

Es können jedoch auch Halogensulfonamide mit gängigen Fluorierungsreagenzien, beispielsweise Antimontrifluorid, Arsentrifluorid oder Kaliumfluorid, umgesetzt werden.

In einer Apparatur mit Rückflußkühler und Rührer werden Halogensulfonamide in geeigneten Lösungsmitteln, beispielsweise Benzol, mit einem Fluorierungsreagenz unter Rühren 1-4 Stunden, vorzugsweise 2 Stunden, unter Rückfluß gekocht. Nach dem Abkühlen auf Raumtemperatur wird die Reaktionslösung filtriert. Das Lösungsmittel wird abdestilliert und der Rückstand unter vermindertem Druck destilliert. Wenn erforderlich wird eine Redestillation unter Normaldruck durchgeführt.

Entflammbarkeit

Es wurde die Entflammbarkeit der Verbindungen der Formel (I) untersucht. Nach den oben dargestellten Verfahren hergestellte Verbindungen sollten mit Hilfe einer offenen Flamme entzündet werden. Die Versuche schlugen fehl.

Elektrochemische Stabilität

Einem Elektrolyt, bestehend aus gängigen Leitsalzen wie $LiPF_6$, $LiBF_4$, $LiClO_4$, $LiAsF_6$, $LiCF_3SO_3$, $LiN(CF_3SO_2)_2$ oder $LiC(CF_3SO_2)_3$ und deren Mischungen in aprotischen Lösungsmitteln wie EC, DMC, PC, DEC, BC, VC, Cyclopentanone, Sulfolane, DMS, 3-Methyl-1,3-oxazolidine-2-on, DMC, DEC, γ-Butyrolacton, EMC, MPC, BMC, EPC, BEC, DPC, 1,2-Diethoxymethan, THF, 2-Methyltetrahydrofuran, 1,3-Dioxolan, Methylacetat, Ethylacetat und deren Mischungen werden Verbindungen der Formel (I) zugesetzt. Der Anteil der Verbindungen der Formel (I) im Lösungsmittelgemisch liegt zwischen 1 und 100%.

In einer Meßzelle mit Edelstahl, Platin- bzw. Goldarbeitselektrode, Lithiumgegenelektrode und Lithiumreferenzelektrode wurden jeweils 3-5 Zyklovoltammogramme hintereinander aufgenommen. Hierzu wurde ausgehend vom Ruhepotential das Potential zuerst mit einer Vorschubgeschwindigkeit von 1 mV/s bis 100 mV/s auf Spannungen größer als das jeweilige Zersetzungspotential des entsprechenden Additivs gegen $Li/Li^+$ erhöht und im weiteren Verlauf zurück auf das Ruhepotential gefahren.

Die Ergebnisse zeigen, daß diese Elektrolyten erst bei Potentialen von ca. 5,0 V gegen $Li/Li^+$ oxidativ zersetzt werden. Damit sind sie für den Einsatz in elektrochemischen Zellen geeignet.

Mischbarkeit mit Standardlösungsmitteln und erhaltene Leitfähigkeiten

Zu einem Referenzelektrolyten, bestehend aus $LiPF_6$, $LiBF_4$, $LiClO_4$, $LiAsF_6$, $LiCF_3SO_3$, $LiN(CF_3SO_2)_2$ oder $LiC(CF_3SO_2)_3$ und deren Mischungen in aprotischen Lösungsmitteln wie EC, DMC, PC, DEC, BC, VC, Cyclopentanone, Sulfolane, DMS, 3-Methyl-1,3-oxazolidine-2-on, DMC, DEC, γ-Butyrolacton, EMC, MPC, BMC, EPC, BEC, DPC, 1,2-Diethoxymethan, THF, 2-Methyltetrahydrofuran, 1,3-Dioxolan, Methylacetat, Ethylacetat und deren Mischungen, wurden portionsweise steigende Mengen von Verbindungen der Formel (I) gegeben.

Es konnten keine Phasentrennungen bzw. Kristallisationen des Leitsalzes beobachtet werden. Die Verbindungen der Formel (I) sind unbegrenzt mit den Referenzelektrolyten mischbar.

Untersuchungen der Leitfähigkeit werden mit einem Referenzelektrolyten bei verschiedenen Temperaturen durchgeführt.

Die nachfolgenden Beispiele sollen die Erfindung näher erläutern, ohne sie jedoch zu beschränken.

Beispiele

Beispiel 1

N,N-Dimethyltrifluormethylsulfonamid

Die Reaktion wird in einem 3-Hals-Kolben mit Kühlfalle, Rührer und Vorrichtung zur Zuführung von gasförmigen Reagenzien durchgeführt. Die Kühlfalle wird bei einer Temperatur von -78°C gehalten. 250 $cm^3$ Diethylether werden im Kolben vorgelegt und mit Eiswasser gekühlt. 138 g (3,07 mol) über Kaliumhydroxid getrocknetes gasförmiges Dimethylamin, erhalten aus der Umsetzung von 260 g (3,19 mol) Dimethylaminhydrochlorid mit 153 g (3,83 mol) gesättigter Natriumhydroxid-Lösung, werden im Kolben kondensiert. 202 g (1,33 mol) gasförmiges Trifluormethylsulfonylfluorid werden über 2 Stunden unter Rühren zugegeben. Nach der Zugabe aller Reagenzien wird das Reaktionsgefäß über 2 Stunden auf 40°C erwärmt. Das Reaktionsgemisch wird mit 0,5 l Wasser verdünnt und anschließend mit Diethylether extrahiert. Das Extrakt wird mit Wasser gewaschen und über $MgSO_4$ getrocknet. Der Diethylether wird abdestilliert und der Rückstand bei Normaldruck destilliert.
230,4 g N, N-Dimethyltrifluormethylsulfonamid werden gewonnen.

Ausbeute: 98%

$CF_3SO_2N(CH_3)_2$: Sdp.: 151-152°C

$^{19}F$-NMR: -75,1 sep ($CF_3SO_2$)

$^{1}H$-NMR: 3,05 q (2 $CH_3$)

$^{5}J_{H,F}$= 1,2 Hz

Beispiel 2

N, N-Dimethylnonafluorbutylsulfonamid

Die Reaktion wird in einem 3-Hals-Kolben mit Kühlfalle, Rührer und Tropftrichter durchgeführt. Die Kühlfalle wird bei einer Temperatur von -78°C gehalten. 100 g (0,331 mol) Perfluorbutylsulfonylfluorid werden langsam unter Rühren zu 43 g (0,95 mol) flüssigem Dimethylamin bei -30°C gegeben. Nach der Zugabe wird die Reaktionsmischung auf Raumtemperatur erwärmt und 3 Stunden gerührt. Es werden 0,1 l Wasser zugegeben und anschließend mit Diethylether extrahiert. Der Extrakt wird mit Wasser gewaschen und über $Na_2SO_4$ getrocknet. Das Lösungsmittel wird abdestilliert.
Es werden 114,5 g weißes kristallines N,N-Dimethylnonafluorbutylsulfonamid gewonnen.

Ausbeute: 87,5 %

$C_4F_9SO_2N(CH_3)_2$, Smp.: 32°C

$^{19}F$-NMR: -81,5 tt (3F, $CF_3$)

-112,2 tm (2F, $CF_2$)

-121,9 m (2F, $CF_2$)

-126,4 tm (2F, $CF_2$)

$^{3}J_{F,F}$=2,2 Hz

$^{4}J_{F,F}$=9,9 Hz

$^{4}J_{F,F}$=13,9 Hz

$^{1}H$-NMR: 3,1 s (2$CH_3$)

Beispiel 3

Bis(N,N-dimethylamidosulfonyl)difluormethan

Die Reaktion wird in einem 3-Hals-Kolben mit Kühlfalle, Rührer und Tropftrichter durchgeführt. Die Kühlfalle wird bei einer Temperatur von -78°C gehalten. 99 g (0,458 mol) Di(fluorsulfonyl)difluormethan werden langsam unter Rühren zu 101 g (2,084 mol) flüssigem Dimethylamin in 100 $cm^3$ Chloroform bei -30°C gegeben. Nach der Zugabe wird die Reaktionsmischung auf Raumtemperatur erwärmt und 3 Stunden gerührt. Das Lösungsmittel wird abdestilliert. Zum Rückstand werden 0,3 l Wasser gegeben und anschließend mit Diethylether extrahiert. Der Extrakt wird mit Wasser gewaschen und über $Na_2SO_4$ getrocknet. Es werden ca. 80% des Lösungsmittels abdestilliert.

Es werden 91,2 g weißes kristallines Bis(N,N-dimethylamidosulfonyl) difluormethan gewonnen.

Ausbeute: 74,8%
$(CH_3)_2NSO_2CF_2SO_2N(CH_3)_2$, Smp.: 71-72°C
$^{19}F$-NMR: -100,4 m (2F, $CF_2$)
$^1H$-NMR: 3,06 t ($4CH_3$)
$^5J_{H,F}$=1,0 Hz

Beispiel 4

N,N-Dimethylamidosulfonylfluorid

Die Reaktion wird in einem 2-Hals-Kolben mit Rührer und Kühlung durchgeführt. 80 g (0,557 mol) N,N-Dimethylamidosulfonylchlorid in 100 $cm^3$ trockenem Benzol werden zu 66 g (0,369 mol) Antimontrifluorid gegeben. Unter Rühren werden 5 $cm^3$ Antimonpentachlorid zugegeben. Das Reaktionsgemisch wird 2 Stunden unter Rückfluß gekocht. Nach dem Abkühlen auf Raumtemperatur wird die Lösung filtriert. Das Benzol wird abdestilliert und der Rückstand unter reduziertem Druck destilliert. Die Redestillation unter Normaldruck ergab 53,8 g reines N,N-Dimethylamidosulfonylfluorid (R. Heap, J.Chem.Soc. 1948, 1313).

Ausbeute: 76%

$FSO_2N(CH_3)_2$, Sdp.: 149-150°C

$^{19}F$-NMR: 33,0 sep

$^4J_{H,F}$=2,0 Hz

Beispiel 5

Entflammbarkeit von N,N-Dimethyltrifluormethylsulfonamid

Mit Hilfe einer offenen Flamme wurde versucht 100 ml N,N-Dimethyltrifluormethylsuifonamid an der Luft zu entzünden. Der Versuch schlug fehl.

Beispiel 6

Elektrochemische Stabilität von N,N-Dimethyltrifluormethylsulfonamid

In einer Meßzelle mit Platinelektrode, Lithiumgegenelektrode und Lithiumreferenzelektrode wurden jeweils 5 Zyklovoltammogramme hintereinander aufgenommen. Hierzu wurde ausgehend vom Ruhepotential das Potential zuerst mit einer Vorschubgeschwindigkeit von 10 mV/s bzw. auf 6 V gegen $Li/Li^+$ erhöht und im weiteren Verlauf zurück auf das Ruhepotential gefahren.

Es zeigt sich der in Abbildung 1 angegebene charakteristische Verlauf. Der Elektrolyt, bestehend aus 1 mol/l $LiPF_6$ in EC/DMC/ N,N-Dimethyltrifluormethylsulfonamid (47,5/47,5/5), wird erst ab einem Potential von ca. 5,5 V gegen $Li/Li^+$ oxidativ zersetzt. Damit ist der Elektrolyt für einen Einsatz in Lithiumionen Batterien mit Übergangsmetallkathode geeignet.

Beispiel 7

Mischbarkeit mit Standardlösungsmitteln und erhaltene Leitfähigkeiten

Zu einem Referenzelektrolyten (1 mol/l $LiPF_6$ in EC/DMC (1:1)) werden portionsweise steigende Mengen N,N-Dimethyltrifluormethylsulfonamid gegeben. Das fluorierte Lösungsmittel ist unbegrenzt mit dem Referenzelektrolyten mischbar. Es konnte keine Phasentrennung bzw. Kristallisation des Leitsalzes beobachtet werden.

Leitfähigkeitsdaten:

Elektrolyt: 1 mol/l $LiPF_6$ in EC/DMC/ N,N-Dimethyltrifluormethylsulfonamid (47,5/47,5/5)

| Temperatur in °C | 20 | -20 | -30 |
|---|---|---|---|
| Leitfähigkeit in mS/cm | 8,6 | 2,9 | 2,1 |

**Patentansprüche**

1. Verbindungen der Formel

$$X\text{-}(CYZ)_m\text{-}SO_2N(CR^1R^2R^3)_2 \qquad (I)$$

mit

| | |
|---|---|
| X | H, F, Cl, $C_nF_{2n+1}$, $C_nF_{2n-1}$, $(SO_2)_kN(CR^1R^2R^3)_2$ |
| Y | H, F, Cl |
| Z | H, F, Cl |
| $R^1$, $R^2$, $R^3$ | H und/oder Alkyl, Fluoralkyl, Cycloalkyl |
| m | 0-9 und falls X=H, $m \neq 0$ |
| n | 1-9 |
| k | 0, falls m=0 und k=1, falls m=1-9. |

2. Verfahren zu Herstellung von Verbindungen gemäß Anspruch 1, dadurch gekennzeichnet, daß teil- oder perfluorierte Alkysulfonylfluoride mit Dimethylamin in organischen Lösungsmitteln umgesetzt werden.

3. Verfahren zur Herstellung von Verbindungen gemäß Anspruch 1, dadurch gekennzeichnet, daß Halogensulfonamid mit gängigen Fluorierungsreagenzien in organischen Lösungsmitteln umgesetzt wird.

4. Verfahren gemäß einem der Ansprüche 2 oder 3, dadurch gekennzeichnet, daß die organischen Lösungsmittel, ausgewählt aus der Gruppe Diethylether, Benzol und Chloroform sind.

5. Verwendung der Verbindungen nach Anspruch 1 als schwer entflammbares Lösungsmittel in Elektrolyten für elektrochemische Zellen.

6. Verwendung nach Anspruch 5, worin besagte Verbindungen zusammen mit anderen gängigen Lösungsmitteln eingesetzt werden.

7. Elektrolyt, dadurch gekennzeichnet, daß er eine oder mehrere Verbindungen gemäß Anspruch 1 enthält.

8. Elektrochemische Zelle bestehend im wesentlichen aus Kathode, Anode, Separator und einem Elektrolyten gemäß Anspruch 7.

9. Lithiumbatterie und Superkondensator gemäß Anspruch 8.

Europäisches Patentamt

# EUROPÄISCHER RECHERCHENBERICHT

Nummer der Anmeldung
EP 00 12 0189

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 4 296 034 A (BOUVET PIERRE ET AL) 20. Oktober 1981 (1981-10-20)<br>* Beispiel 8 * | 1-4 | C07C311/09<br>C07C303/38 |
| X | US 4 921 696 A (LOFGREN CLIFFORD S ET AL) 1. Mai 1990 (1990-05-01)<br>* Spalte 9; Beispiel 10707; Tabelle 1 * | 1 | |
| X | SARTORI P ET AL: "The actual state of our knowledge about mechanism of electrochemical fluorination in anhydrous hydrogen fluoride (Simons process)" JOURNAL OF FLUORINE CHEMISTRY,CH,ELSEVIER SEQUOIA. LAUSANNE, Bd. 87, Nr. 2, 2. Februar 1998 (1998-02-02), Seiten 157-162, XP004111460 ISSN: 0022-1139<br>* seite 158, verbindungen * | 1 | |
| X | NETSCHER T ET AL: "Formation of Sulfinate Esters in the Synthesis of Triflates" TETRAHEDRON LETTERS,NL,ELSEVIER SCIENCE PUBLISHERS, AMSTERDAM, Bd. 37, Nr. 46, 11. November 1996 (1996-11-11), Seiten 8359-8362, XP004068662 ISSN: 0040-4039<br>* scheme 1, struktur 13 * | 1 | RECHERCHIERTE SACHGEBIETE (Int.Cl.7)<br>C07C |
| | -/-- | | |

Der vorliegende Recherchenbericht wurde für alle Patentansprüche erstellt

| Recherchenort | Abschlußdatum der Recherche | Prüfer |
|---|---|---|
| BERLIN | 30. Oktober 2000 | O'Sullivan, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

**EUROPÄISCHER RECHERCHENBERICHT**

Nummer der Anmeldung
EP 00 12 0189

**EINSCHLÄGIGE DOKUMENTE**

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | CHEMICAL ABSTRACTS, vol. 109, no. 11, 12. September 1988 (1988-09-12) Columbus, Ohio, US; abstract no. 88123v, TAMARU, MASATOSHI ET AL: "Structure-activity study of systemic acaricidal N-substituted chloromethanesulfonamides by the ORMUCS method" Seite 257; Spalte 1; XP002151387 * Zusammenfassung * & NIPPON NOYAKU GAKKAISHI, Bd. 13, Nr. 1, 1988, Seiten 1-6, --- | 1 | |
| X | CHEMICAL ABSTRACTS, vol. 79, no. 17, 29. Oktober 1973 (1973-10-29) Columbus, Ohio, US; abstract no. 104719p, DIBROVA, A K ET AL : "Fluorine-containing beta-sultones. 41. alpha-Hydrotetrafluoroethanesulfonic acid amides." Seite 385; Spalte 2; XP002151388 * Zusammenfassung * & IZV. AKAD. NAUK SSSR, SER. KHIM, Bd. 7, 1973, Seiten 1563-7, --- | 1-4 | RECHERCHIERTE SACHGEBIETE (Int.Cl.7) |
| | -/-- | | |

Der vorliegende Recherchenbericht wurde für alle Patentansprüche erstellt

| Recherchenort | Abschlußdatum der Recherche | Prüfer |
|---|---|---|
| BERLIN | 30. Oktober 2000 | O'Sullivan, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 00 12 0189

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | CHEMICAL ABSTRACTS, vol. 88, no. 25, 19. Juni 1978 (1978-06-19) Columbus, Ohio, US; abstract no. 190015w, RADCHENCKO, O A ET AL: "Isoperfluoroalkanesulfinic and -sulfonic acids. I. Heptafluoropropane-2-sulfinic and -sulfonic acids." Seite 694; Spalte 2; XP002151389 * Zusammenfassung * & ZH. ORG. KHIM., Bd. 14, Nr. 2, 1978, Seiten 275-8, --- | 1 | |
| X | CHEMICAL ABSTRACTS, vol. 88, no. 5, 30. Januar 1978 (1978-01-30) Columbus, Ohio, US; abstract no. 37193h, GLASS, R S ET AL: "Preparation of trichloromethanesulfonamides via trichloromethanesulfenamides." Seite 452; Spalte 1; XP002151390 * Zusammenfassung * & SYNTHESIS., Nr. 11, 1977, Seiten 798-800, GEORG THIEME VERLAG. STUTTGART., DE ISSN: 0039-7881 --- -/-- | 1 | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| BERLIN | 30. Oktober 2000 | O'Sullivan, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 00 12 0189

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | GLASS, R S ET AL : "Nucleophilic substitution reactions of N-alkyldi(trifluoromethane)sulfonamides. Role of the solvent hexamethylphosphoric triamide" JOURNAL OF ORGANIC CHEMISTRY., Bd. 43, Nr. 11, 1978, Seiten 2291-4, XP002151384 AMERICAN CHEMICAL SOCIETY. EASTON., US ISSN: 0022-3263 * Beispiel 1A; Tabelle II * | 1 | |
| X | EDWARDS, M L ET AL : "Stereospecific synthesis of secondary amines by the Mitsunobu reaction" TETRAHEDRON LETTERS., Bd. 31, Nr. 24, 1990, Seiten 3417-20, XP002151385 ELSEVIER SCIENCE PUBLISHERS, AMSTERDAM., NL ISSN: 0040-4039 * scheme 2, struktur 21,26 und 27* | 1-4 | RECHERCHIERTE SACHGEBIETE (Int.Cl.7) |
| X | BEYER, H; WALTER, W: "Lehrbuch der organischen chemie" 1988 , S HIRZEL VERLAG , STUTTGART XP002151386 * seite 496, 5.4.2.1 "sulfonamide" * | 2-4 | |
| A | WO 99 05100 A (ARMAND MICHEL ;UNIV MONTREAL (CA); ACEP INC (CA); MICHOT CHRISTOPH) 4. Februar 1999 (1999-02-04) * Zusammenfassung * * Ansprüche 1,27,32-34 * | 1-9 | |

-/--

Der vorliegende Recherchenbericht wurde für alle Patentansprüche erstellt

| Recherchenort | Abschlußdatum der Recherche | Prüfer |
|---|---|---|
| BERLIN | 30. Oktober 2000 | O'Sullivan, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 00 12 0189

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | US 4 074 028 A (WILL FRITZ G) 14. Februar 1978 (1978-02-14) * Spalte 1, Zeile 60 - Spalte 2, Zeile 4 * * Spalte 4, Zeile 51 - Spalte 5, Zeile 17 * | 1-9 | |
| A | EP 0 902 492 A (HYDRO QUEBEC) 17. März 1999 (1999-03-17) * Spalte 2, Zeile 15 - Zeile 45 * * Beispiele * | 1-9 | |
| | | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| BERLIN | 30. Oktober 2000 | O'Sullivan, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 00 12 0189

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-10-2000

| Im Recherchenbericht angeführtes Patentdokument | Datum der Veröffentlichung | Mitglied(er) der Patentfamilie | Datum der Veröffentlichung |
|---|---|---|---|
| US 4296034 A | 20-10-1981 | FR 2029262 A | 16-10-1970 |
| | | BE 744213 A | 15-06-1970 |
| | | DE 2002460 A | 30-07-1970 |
| | | GB 1309831 A | 14-03-1973 |
| | | NL 6919279 A | 24-07-1970 |
| US 4921696 A | 01-05-1990 | US 5177107 A | 05-01-1993 |
| | | US 5364618 A | 15-11-1994 |
| | | AU 582533 B | 06-04-1989 |
| | | AU 2492384 A | 02-08-1984 |
| | | BR 8404556 A | 11-12-1984 |
| | | JP 2780913 B | 30-07-1998 |
| | | JP 7173013 A | 11-07-1995 |
| | | JP 6035365 B | 11-05-1994 |
| | | JP 60500500 T | 11-04-1985 |
| | | WO 8402650 A | 19-07-1984 |
| | | BR 8301452 A | 29-11-1983 |
| WO 9905100 A | 04-02-1999 | EP 0928287 A | 14-07-1999 |
| US 4074028 A | 14-02-1978 | KEINE | |
| EP 0902492 A | 17-03-1999 | CA 2215849 A | 11-03-1999 |
| | | CA 2246955 A | 11-03-1999 |
| | | JP 11171853 A | 29-06-1999 |

EPO FORM P0461

Für nähere Einzelheiten zu diesem Anhang : siehe Amtsblatt des Europäischen Patentamts, Nr.12/82